# EUROPEAN PATENT APPLICATION

(11) **EP 4 623 824 A1**
(43) Date of publication of application: **01.10.2025**
(21) Application number: 24167429.0
(22) Date of filing: 28.03.2024
(51) Int. Cl.: A61B 5/257, A61B 5/00

(54) **HOLDER APPARATUS FOR PATCH ELECTRODE STRUCTURE AND BIO-SIGNAL PROCESSING DEVICE AND ITS FABRICATION METHOD**

(71) Applicant: Bittium Biosignals Oy, 70800 Kuopio (FI)
(72) Inventor: Nikula, Arto, 70800 Kuopio (FI); Myllykangas, Juha, 70800 Kuopio (FI); Perälä, Tommi, 70800 Kuopio (FI); Halonen, Jani, 70800 Kuopio (FI)
(74) Representative: Kolster Oy Ab

(57) **Abstract**

A holder apparatus (100) for a patch electrode structure (200) and a bio-signal processing device (300) comprises a first front and rear recesses (1040, 1042) of a first polymer cover part (104), the first front recess (1040) fitting to the bio-signal device (300). The first cover part (104) is attached with a double-sided adhesive sheet (110). An electric connector part (106) comprises a connector (1060) for the bio-signal processing device (300), and a flexible conductor cable (1064) that connects with a connector (1060) and holder electrodes (1066) of a support sheet (1062). The flexible conductor cable (1064) and/or the connector (1060) extend through a gap (1052) and a first aperture (1050) of the first cover part (104). An adapter component (108) is between the support sheet (1062) and the first cover part (104) for support of the connector (1060) and fills the first aperture (1050) of the first cover part (104). The first cover part (104) and the first front recess (1040) form a pocket (102A) for the bio-signal processing device (300).

## Description

### Field

The invention relates to a holder apparatus for a patch electrode structure and a bio-signal processing device.

### Background

An electronic device, which measures bio-signals such as ECG (ElectroCardioGram) and EEG (ElectroEncephaloGram), must be well contacted with the electrodes that are in contact with the body and mechanically reliably fixed to its support.

A bio-signal processing device can be inserted in and removed from a holder, which may be made of polymer. The holder includes an electrically conductive contact structure at its rear section for having an electric contact with electrodes and/or sensors that measure the bio-signals from the body.

A construct and final assembly of this kind of holder with the electrically conductive contact structure for disposable electrodes should be simple, save energy and material(s), have a low number of phases of assembly and keep assembly cost at minimum.

A typical prior art solution is to use insertion injection molding with separate gaskets, PCB's, screws, assembly jigs and manual labor to make and put together the parts. The holder may be over-molded with the electrically conductive contact structure, by pouring liquid into the assembly that is kept at a correct position by assembly jigs. These processes are slow, costly and problems with yield are inevitable. The prior art solutions also use contradictious manufacturing processes that may affect positively at some stage but negatively at other stages of manufacturing process. Hence, an improvement would be welcome.

### Brief description

The present invention seeks to provide an improvement in the structure and the assembly.

The invention is defined by the independent claims. Embodiments are defined in the dependent claims.

If one or more of the embodiments is considered not to fall under the scope of the independent claims, such an embodiment is or such embodiments are still useful for understanding features of the invention.

### List of drawings

Example embodiments of the present invention are described below, by way of example only, with reference to the accompanying drawings, in which
Figure 1A illustrates an example of an exploded-view of a holder apparatus;
Figure 1B illustrates an example of a double-sided adhesive sheet;
Figure 2 illustrates an example of a perspective view from a backside of the holder apparatus;
Figure 3 illustrates an example of the holder apparatus and a patch electrode structure;
Figure 4 illustrates an example of a bio-signal processing device being about to be inserted into a pocket of the holder apparatus;
Figure 5 illustrates an example of a flow chart of a method of fabricating a bio-signal arrangement; and
Figure 6 illustrates of an example of a flow chart of the method of fabricating a bio-signal arrangement with two cover parts.

### Description of embodiments

The following embodiments are only examples. Although the specification may refer to "an" embodiment in several locations, this does not necessarily mean that each such reference is to the same embodiment(s), or that the feature only applies to a single embodiment.

Single features of different embodiments may also be combined to provide other embodiments. Furthermore, words "comprising" and "including" should be understood as not limiting the described embodiments to consist of only those features that have been mentioned and such embodiments may also contain features/structures that have not been specifically mentioned. All combinations of the embodiments are considered possible if their combination does not lead to structural or logical contradiction.

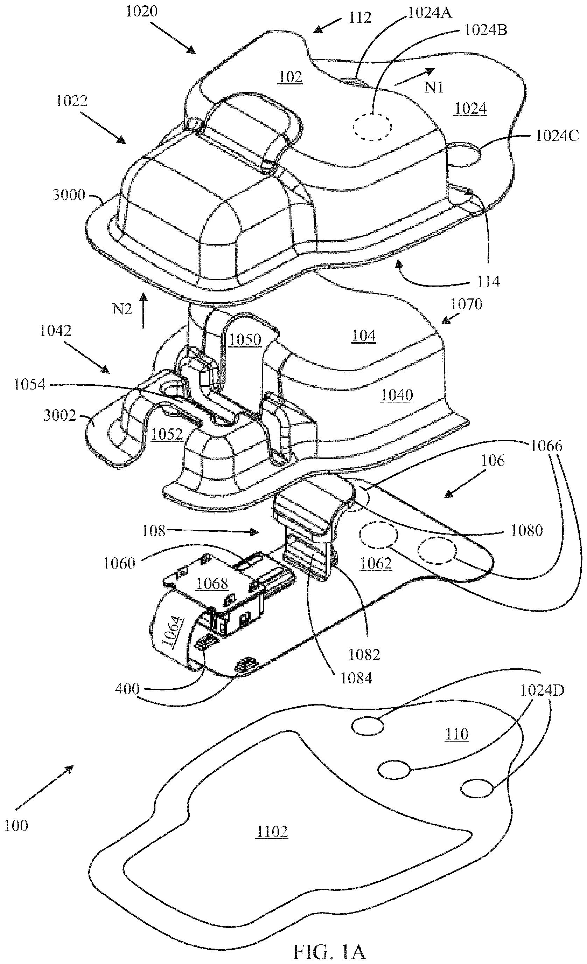

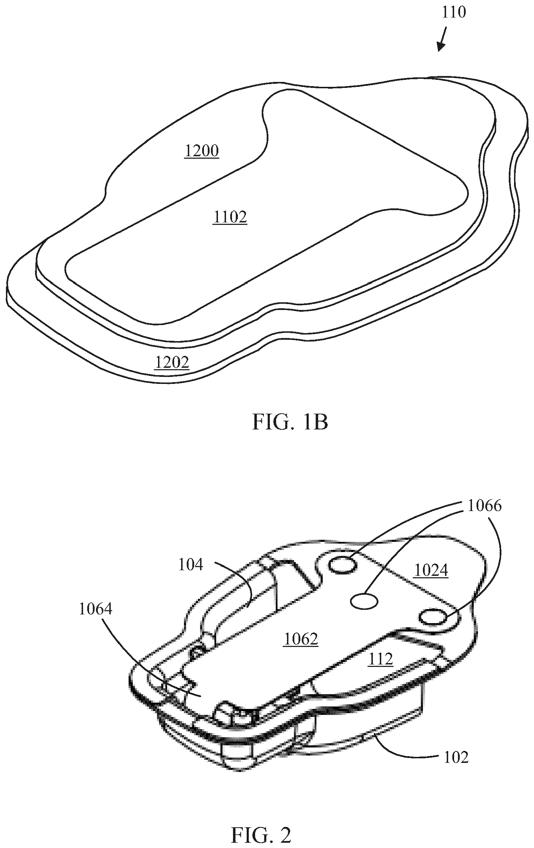

Fig. 1 illustrates a holder apparatus 100 for a patch electrode structure 200 and a bio-signal processing device 300. The holder apparatus 100 comprises a first cover part 104 comprising a sheet curved in a form that has a recess structure. The first cover part 104 is a continuous sheet of material that has the recess structure which, in turn, can be considered a depression or an indentation. The concave side of the recess is open. The recess structure has a first front recess 1040 and a first rear recess 1042. The first cover structure 104 may be formed using vacuum forming. In the vacuum forming a sheet or layer is placed on the mold. Then a suction force with potential heating is applied to the mold, the suction bending the sheet and forcing it against the walls of the mold. That means that the sheet will have the same recess shape as the mold, which in turn has the desired shape corresponding to that of the bio-signal device 300. Vacuum forming is a simple way to thermoform plastic or polymer sheets. Instead of vacuum forming, the first cover part 104 may be formed by injection molding, for example. Injection molding is a thermoplastic process where melt polymer is mechanically forced into a shape of a mold that has the desired recess shape.

The first front recess 1040 can receive the bio-signal device 300 and the first front recess 1040 and an outer contour of the bio-signal device 300 fit accurately together. That means, the first front recess 1040 is of a proper size and shape for the bio-signal device 300. When the bio-signal device 300 is in the first front recess 1040, the bio-signal device 300 and the first front recess 1040 for a friction contract to each other such that some force is required to remove the bio-signal device 300 from the first front recess 1040. The force required for insertion of the bio-signal processing device 300 to the first front recess 1040 and removal of the bio-signal device 300 from the first front recess 1040, however, does not require a usage of tools. That means the insertion and removal can be performed in a tool-less manner using fingers only. A person with a normal finger force can insert the bio-signal processing device 300 into the first front recess 1040 and he/she can also remove the bio-signal processing device 300 from the first front recess 1040.

The first cover part 104 comprises a first hole structure at a rear part of the recess structure. The hole structure may comprise an aperture 1050 at a rear part of the front recess 1040 and a gap 1052 at a rear part of the rear recess 1042.

The first cover part 104 is attached with a double-sided adhesive sheet 110 such that the concave side is facing the double-sided adhesive sheet 110.

The first cover part 104 has a front side of the first front recess 1040 open, and the first cover part 104 and the double-sided adhesive sheet 110 enclose a fifth aperture 1070 of the holder apparatus 100 when the first cover part 104 is attached with the patch electrode structure 200. In this manner, a continuous material strip of the adhesive sheet 110 is a link between opposite sides of the first cover part 104.

The holder apparatus 100 further comprises an electric connector part 106 that comprises a connector 1060 for the bio-signal processing device 300, a support sheet 1062 and a flexible conductor cable 1064. The flexible conductor cable 1064 connects the connector 1060 and holder electrodes 1066 of the support sheet 1062. The flexible conductor cable 1064 and/or the connector 1060 being configured to extend through the gap 1052 and the first aperture 1050 of the first cover part 104. In an embodiment, the connector 1060 may comprise a male connector an example of which is a USB-connector (Universal Series Bus connector). In an embodiment, the USB-connector may be a male micro-USB connector. The connector 1060 may also comprise a piece of a printed circuit board 1068. The bio-signal device 300 has then a corresponding counter-connector.

The rear recess 1042 of the first cover part 104 that has a cavity for the connector 1060 may be filled with adhesive. In the embodiment, where both the first and second cover parts 104, 102 are used, the cavities formed by the rear recesses 1042, 1022 may be filled with adhesive. The adhesive may be optically curable, for example. The filling with adhesive may be performed automatically.

The holder apparatus 100 comprises an adapter component 108 that is in contact with the support sheet 1062 and the connector 1060. The adapter component 108 is between the support sheet 1062 and the first cover part 104 for support of the connector 1060. Additionally, the adapter component 108 fills the first aperture 1050 of the first cover part 104 together with the flexible cable 1064 and/or the connector 1060 of which at least one continues materialistically from one side to another side of the first aperture 1050.

The adapter component 108 is a mechanical structure that fills the first aperture 1050 of the first cover part 104. It may prevent sweat or in general moisture from entering in the space formed by the first recess 1040 of the first cover part 104 and a patch electrode structure 200 attaches with it. The adapter component 108 may be elastic and/or flexible.

The fifth aperture 1070 of the first cover part 104, and the first front recess 1040 of the first cover part 104 forms a pocket 102A for the bio-signal processing device 300 to be inserted into and/or removed from. The insertion into and removal from the pocket 102A can be performed in a tool-less manner using fingers only. A person with a normal finger force can insert the bio-signal processing device 300 into the pocket 102A and he/she can also remove the bio-signal processing device 300 from the pocket 102A.

In an embodiment, the holder apparatus may comprise a second cover part 102 comprising a sheet curved in the form of a second front recess 1020 and a second rear recess 1022. The second recess structure is formed in a manner to one of those mentioned in the conjunction of the first recess structure.

A concave side of the second front recess 1020 is made to fit to a convex side of the first front recess 1040 of the first cover part 104.

The second cover part 102 comprises a third aperture 114 at a bottom side of the second recess structure front and rear recesses 1020, 1022, and a fourth aperture 112 at front side of the second front recess 1020. The third and fourth apertures 112, 114 are directed in orthogonal directions, and they are separated by a continuous material strip 1024 of the second cover part 102. Normal N1 of the fourth aperture 112 and the normal N2 of the third aperture 114 being at least approximately orthogonal. One of the normal is pointing down and another of the normal is pointing horizontally.

The second cover part 102 and the double-sided adhesive sheet 110 are attached together by the adhesive of the adhesive sheet 110.

The second cover part 102 is configured to receive the first cover part 104 through the third aperture 114 of the second cover part 102.

The second cover part 102 being configured to press the first cover part 104 toward the double-sided adhesive sheet 110 in conjunction with attachment of the second cover part 102 and the double-sided adhesive sheet 110. This arrangement makes the holder apparatus a compact baggage.

The fourth aperture 112 of the second cover part 102, the fifth aperture 1070 of the first cover part 104, the first front recess 1040 of the first cover part 104 and the second front recess 1020 of the second cover part 102 are configured to form a pocket 102A for the bio-signal processing device 300 to be inserted into and/or removed from.

Note that the double-sided adhesive sheet 110 may be an independent component of the holder apparatus 100 that may be attached first to either of the holder apparatus or the patch electrode structure 200. Alternatively, it may be a part of either of the holder apparatus 100 or the patch electrode apparatus 200.

In an embodiment, the continuous material strip 1024 of the second cover part 102 and the double-sided adhesive sheet 110 are attached together based on adhesive of the double-sided adhesive sheet 110. In an embodiment, the continuous material strip 1024 may have holes 1024A, 1024B, 1024C at positions and of sizes corresponding to those of contact electrodes 2002 of the patch electrode structure 200. The holes 1024A, 1024B and 1024C can be used when the holder electrodes 1066 of the support sheet 1062 and the contact electrodes 2002 of the patch electrode structure 200 are pressed together during a fabrication phase. The double-sided adhesive sheet 110 may have corresponding hole 1024B. The holes 1024A, 1024B, 1024C and 1024D enable the pressure to be directed through the holes 1024A, 1024B and 1024C to the electrodes 1066 and 1002 without causing pressure to other structures of the holder apparatus 100 and the patch electrode structure 200. The pressure may be applied by pinchers or the like.

In an embodiment, the support sheet 1062 may comprise patient protection circuitry resistors 400 of which are shown in Fig. 1A. The circuitry is IEC-approved and tested. The patch electrode structure 200 may in this manner be independent from the holder apparatus 100 without the burden of these requirements. The resistors 400, the number of which may be between 2 to 5 without restricting to these values, consume reduce the energy of an associated device.

Namely, it is possible that in an emergency situation a patient is attempted to defibrate without removing the holder apparatus and the patch electrode structure. IEC60601-1 defines what is sufficient protection against exposure to defibrillation pulse. When a defibrillation voltage is applied to the thorax of a patient, via externally applied paddles/defibrillation electrodes, the body tissue of the patient in the vicinity of the paddles and between the paddles becomes a voltage dividing system with associated devices.

For the energy reduction test, energy (joules) consumed to 100 Ω resistor is set as reference. Energy (joules) consumed to applied part/ ECG device should be less than 10% of initial reference value when surge pulse applied.

The minimum value of the patient protection circuitry resistors 400 is selected based on a shunt energy requirement as well as minimum 4.0 mm creepage distance. Electric field from defibrillation pulse is somewhere between fully homogenous and fully in-homogenous field across the resistor 400. Electrode shunt resistor design and pad dimensions are based on IEC 60664-1 low voltage standard rationales. IEC 60664-1 - standard covers Insulation coordination for equipment within low-voltage supply systems. Selected shunt resistors 400 are designed to withstand medical defibrillator surges as well as to limit surge energy to acceptable level, no safety based design rules are utilized after shunt.

In an embodiment, the double-sided adhesive sheet 110 may comprise a second aperture 1102 at an area of the first front recess 1040. As illustrated in Fig 1B, the support sheet 1062 may be configured to fit inside the second aperture 1102 of the double-sided adhesive sheet 110.

In an embodiment an example which is illustrated in Fig. 1B, the double-sided adhesive sheet 100 may comprise at least two sheet layers 1200, 1202. A first sheet layer 1200 of the two sheet layers may be in contact with the first cover part 104, and a second sheet layer 1202 of the sheet layers, which may be larger than the first sheet layer 1200, may be in contact with the second cover part 102. The sizes of the sheet layers 1200, 1202 may be matched with the edges of the cover parts 102, 104. The edges of the cover parts 102, 104 may be bent such that the recesses of the first and second cover parts 104, 102 have brims 3000, 3002. The brims 3000, 3002 may be put in contact with the double-sided adhesive sheet 110, and in more detail, with the first and second sheet layers 1200, 1202.

In an embodiment, the fifth aperture 1070 of the first cover part 104 may be open toward a direction parallel to that of the first aperture 1050 of the first cover part 104.

In an embodiment, the first front recess 1040 of the first cover part 104 may fit to the outer contour of the bio-signal processing device 300 and cause a frictional contact with the bio-signal processing device 300. In that manner, the bio-signal processing device 300 stays steadily in the pocket 102A when a person carrying the holder apparatus 100 with the bio-signal processing device 300 in the pocket 102 is running or otherwise moving, for example.

In an embodiment, the holder apparatus 100 may comprise adhesive material that causes attachment of at least two of the second cover part 102, the first cover part 104, the electric connector part 106, the adapter component 108 and the double-sided adhesive sheet 110 together. The adhesive material may be cured with optical radiation, while the first cover part 104 and the second cover part 102 are transparent to the optical radiation for the attachment. The adhesive material may be spread between at least two structural parts of the second cover part 102, the first cover part 104, the electric connector part 106, the adapter component 108 and the double-sided adhesive sheet 110. The adhesive material may also be called glue. The adhesive material may be synthetic or natural. The adhesive material layer bonds parts of the holder apparatus 100 together. Additionally, the adhesive material layer may seal gaps between parts of the holder apparatus 100.

In an embodiment, the first cover part 104 may comprise a continuous material bridge 1054 between the first aperture 1050 of the first cover part 104 and the gap 1052. The flexible cable 1064 may be set to continue from the support sheet 1062 attached with the double-sided adhesive sheet 110 though the gap 1052, over the material bridge 1054 toward the first aperture 1050 for a connection with the connector 1060. The flexible cable 1064 and the connector 1060 may be partly inside the adapter component 108.

Fig. 2 illustrates the holder apparatus 100 from below. The support sheet 1062 extends from the rear part of the holder apparatus 100 to the front part where the support sheet 1062 is attached with the continuous material strip 1024 of the second cover part 102. If only one cover part is used, i.e. the first cover part 104, then the first cover part 104 may comprise a continuous material strip similar to the continuous material strip 1024 of the second cover part 102. The support sheet 1062 may be attached to said material strip.

In an embodiment, the adapter component 108 may comprise a hole 1084 that extends through the adapter component 108. The connector 1060 may be partly inside the hole 1084. The adapter component 108 may connect and seal the connector 1060 and the first cover part 104 together.

In an embodiment, material of at least one of the second cover part 102, the first cover part 104, the electric connector part 106, and the adapter component 108 may be made of bio-fiber based polymer. The bio-fiber based polymer may also be called bioplastic, bio-composite and/or biodegradable polymer. Bioplastic is plastic that has biological substances as a raw material. In that manner, petrochemical substances can be reduced or avoided in its production. Bio-composite comprises renewable plastic and fillers such as fibers that also may be natural fibers. Biodegradable plastics are plastics that can be decomposed by the action of living organisms, usually microbes, into water, carbon dioxide, and biomass.

In an embodiment, the adapter component 108 may comprise a mating structure 1080 and a leg structure 1082. The leg structure 1082 may support the mating structure 1080. The leg structure 1082 may comprise a fifth hole 1084 that may receive the connector part 106. The fifth hole 1084 may then keep the connector 1060 at least partly, and the leg structure 1082 supports the connector 1060. The mating structure 1080 may fill the first aperture 1050 of the first cover part 104.

The adapter component 108 may have contact with the second cover part 102 through the first aperture 1050. In that manner, the holder apparatus 100 may be made solidly built and the structural parts can be adjusted at steady and firm locations.

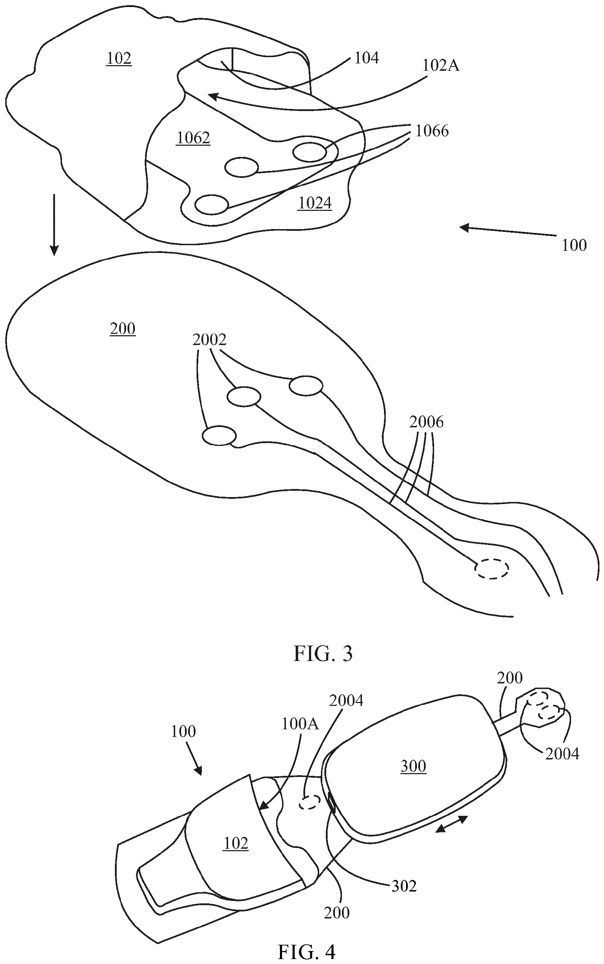

Fig. 3 illustrates an example where the holder apparatus 100 is attached with the patch electrode structure 200. The patch electrode structure 200 comprises contact electrodes 2002, skin electrodes 2004 and electric conductors 2006 between them, the contact electrodes 2002 being configured to be in electric contact with the holder electrodes 1066 of the support sheet 1062 in response to attachment between the holder apparatus 100 and the patch electrode structure 200 with the double-sided adhesive sheet 110.

The patch electrode structure 200 may be fabricated in roll-to-roll process because the patch electrode structure 200 can be fabricated separately from the holder apparatus 100.

Fig. 4 illustrates an example of the bio-signal processing device 300 that is about be inserted into the pocket 102 or has just been taken out from the pocket 102A. The bio-signal processing device 300 may be an electronic device which may convert an analog bio-signal it receives to a digital bio-signal. The bio-signal processing device 300 may also filter the bio-signal in the analog or in the digital form. Additionally or alternatively, the bio-signal processing device 300 may perform data processing of the bio-signal, and it may also store data of the bio-signal and/or a result of its processing. The bio-signal may be related to body movement, body temperature, heart rate variability, electrocardiogram, electromyogram, electroencephalogram or the like for example. The bio-signal processing device 300 is a computer with a computer program suitable for bio-signal processing.

The term "computer" includes a computational device that performs logical and arithmetic operations. For example, a "computer" may comprise an electronic computational device, such as an integrated circuit, a microprocessor, a mobile computing device, a laptop computer, a tablet computer, a personal computer, or a mainframe computer. A "computer" may comprise a central processing unit, an ALU (arithmetic logic unit), a memory unit, and a control unit that controls actions of other components of the computer so that steps of a computer program are executed in a desired sequence. A "computer" may also include at least one peripheral unit that may include an auxiliary memory (such as a disk drive or flash memory), and/or may include data processing circuitry.

The bio-signal processing device may be connected with a user interface, which means an input/output device and/or unit. Non-limiting examples of a user interface include a touch screen, other electronic display screen, keyboard, mouse, microphone, handheld electronic game controller, digital stylus, display screen, speaker, and/or projector for projecting a visual display. In an embodiment, the bio-signal processing device 300 may comprise some kind of user interface such as a screen and/or a touch screen.

In an embodiment, polymer material used for the holder apparatus 100 may be polyethylene terephthalate glycol-modified (PET-G), and/or polypropylene, for example.

This document presents unique design for connecting wearable, disposable accessory part to the bio-signal processing device 300 device that is not disposable. This document presents a simple and potentially waterproof connection between these two devices and offer way to produce cheap and reliable disposable accessories to the non-disposable bio-signal processing device 300.

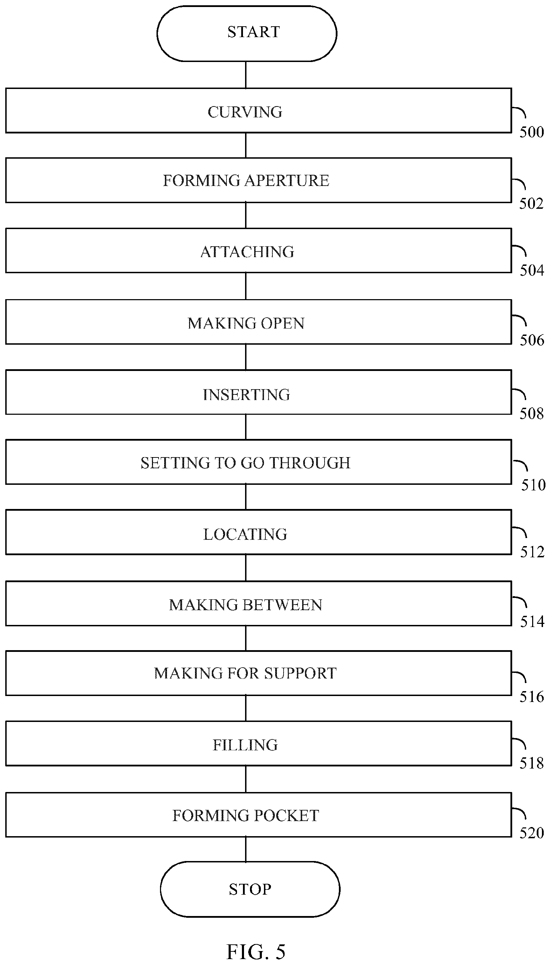

Figure 5 is a flow chart of the method of fabricating a bio-signal arrangement. In step 500, a first cover part 104 is formed by curving a sheet in a form of a first front recess 1040 and a first rear recess 1042, the first front recess 1040 fitting an outer contour of the bio-signal device 300.

In step forming 502, a first aperture 1050 is formed to the first cover part 104 at a rear part of the front recess 1040 and a gap 1052 at a rear part of the rear recess 1042.

In step 504, the first cover part 104 and a double-sided adhesive sheet 110 are attached together such that the concave side is facing the double-sided adhesive sheet 110.

In step 506, a front side of the first front recess 1040 of the first cover part 104 is made open by enclosing a fifth aperture 1070 by the first cover part 104 and the double-sided adhesive sheet 110 in response to attachment of the first cover part 104 and the patch electrode structure 200 together.

In step 508, a connector 1060 that has flexible conductor cable 1064 is inserted through the first aperture 1050 of the first cover part 104.

In step 510, the flexible cable 1064 is set to go through the gap 1052.

In step 512, a support sheet 1062 that has holder electrodes 1066 connected with the flexible cable 1064 is located under the concave first cover part 104.

In step 514, a mechanical contact is made between the support sheet 1062 and the connector 1060 by an adapter component 108.

In step 516, a mechanical contact is made between the first cover part 104 and the connector 1060 by the adapter component 108 for support of the connector 1060.

In step 518, the first aperture 1050 is filled with the adapter component 108, the flexible cable 1064 and/or the connector 1060.

In step 520, a pocket 102A is formed by the fifth aperture 1070 of the first cover part 104, and the first front recess 1040 of the first cover part 104 for the bio-signal processing device 300 to be inserted into and/or removed from.

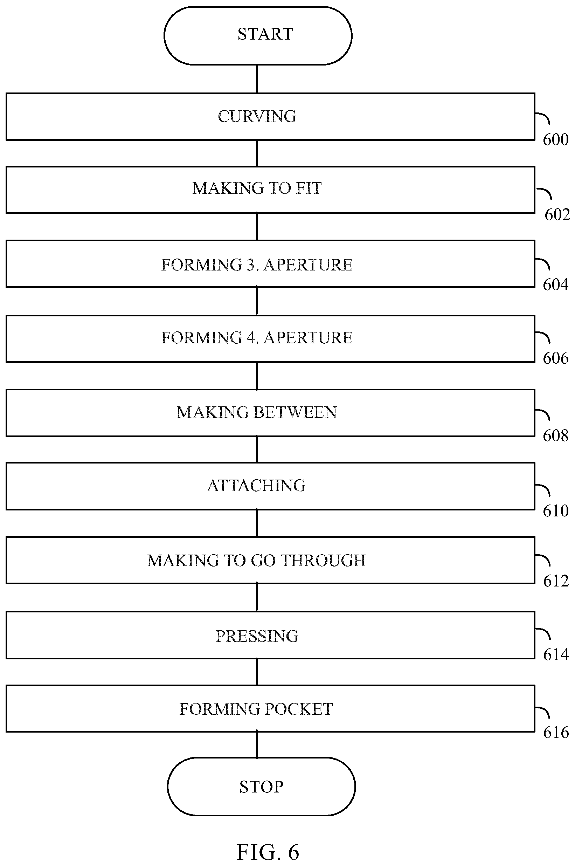

Figure 6 is a flow chart of the method of fabricating a bio-signal arrangement that has two cover parts. In step 600, a second cover part 102 is formed by curving a sheet in a form of a second front recess 1020 and a second rear recess 1022.

In step 602, a concave side of the second front recess 1020 of the second cover part 102 is made to fit to a convex side of the first front recess 1040 of the first cover part 104.

In step 604, a third aperture 114 is formed at a bottom side of the second front and rear recesses 1020, 1022 of the second cover part 102.

In step 606, a fourth aperture 112 is formed at front side of the second front recess 1020 of the second cover part 102, the third and fourth apertures 112, 114 are directed in orthogonal directions, and the third and fourth apertures 112, 114 are separated by a continuous material strip 1024 of the second cover part 102.

In step 608, a mechanical contact is made between the adapter component 108 and the second cover part 102 through the first aperture 1050 of the first cover part 104.

In step 610, attachment of the second cover part 102 and the double-sided adhesive sheet 110 together is performed.

In step 612, the first cover part 104 is made to go through the third aperture 114 of the second cover part 102 in conjunction with the attachment.

In step 614, the first cover part 102 is pressed by the second cover part 102 in conjunction with the attachment.

In step 616, a pocket 102A is formed for the bio-signal processing device 300 to be inserted into and/or removed from by the fourth aperture 112 of the second cover part 102, the fifth aperture 1070 of the first cover part 104, the first front recess 1040 of the first cover part 104 and the second front recess 1020 of the second cover part 102.

The presented solution may create low-cost, waterproof, and simple structure for bio-signal device arrangement. It also is a promising option to be used for existing bio-signal products that are mass produced.

It will be obvious to a person skilled in the art that, as technology advances, the inventive concept can be implemented in various ways. The invention and its embodiments are not limited to the example embodiments described above but may vary within the scope of the claims.

## Claims

1. A holder apparatus (100) for a patch electrode structure (200) and a bio-signal processing device (300), **characterized in that** the holder apparatus (100) comprises
a first cover part (104) comprising a polymer sheet curved in a form of a first front recess (1040) and a first rear recess (1042),
the first front recess (1040) being configured to fit to an outer contour of the bio-signal device (300),
the first cover part (104) comprising a first aperture (1050) at a rear part of the front recess (1040) and a gap (1052) at a rear part of the rear recess (1042),
the first cover part (104) being attached with a double-sided adhesive sheet (110) such that the concave side is facing the double-sided adhesive sheet (110),
the first cover part (104) having a front side of the first front recess (1040) open, and the first cover part (104) and the double-sided adhesive sheet (110) being configured to enclose a fifth aperture (1070) of the holder apparatus (100) when the first cover part (104) is attached with the patch electrode structure (200),
the holder apparatus (100) further comprises an electric connector part (106) that comprises a connector (1060) for the bio-signal processing device (300), a support sheet (1062) and a flexible conductor cable (1064) that is configured to connect the connector (1060) and holder electrodes (1066) of the support sheet (1062), and the flexible conductor cable (1064) and/or the connector (1060) being configured to extend through the gap (1052) and the first aperture (1050) of the first cover part (104);
the holder apparatus (100) still further comprises an adapter component (108) that is configured to be in contact with the support sheet (1062) and the connector (1060) between the support sheet (1062) and the first cover part (104) for support of the connector (1060), and fill the first aperture (1050) of the first cover part (104) together with the flexible cable (1064) and/or the connector (1060);
the fifth aperture (1070) of the first cover part (104), and the first front recess (1040) of the first cover part (104) are configured to form a pocket (102A) for the bio-signal processing device (300) to be inserted into and/or removed from.

2. The holder apparatus of claim 1, **characterized in that** holder apparatus comprises a second cover part (102) comprising a polymer sheet curved in a form of a second front recess (1020) and a second rear recess (1022);
a concave side of the second front recess (1020) being configured to fit to a convex side of the first front recess (1040) of the first cover part (104);
the second cover part (102) comprising a third aperture (114) at a bottom side of the second front and rear recesses (1020, 1022), and a fourth aperture (112) at front side of the second front recess (1020), the third and fourth apertures (112, 114) being directed in orthogonal directions and separated by a continuous material strip (1024) of the second cover part (102);
the second cover part (102) and the double-sided adhesive sheet (110) being configured to be attached together;
the second cover part (102) being configured to receive the first cover part (104) through the third aperture (114) of the second cover part (102);
the second cover part (102) being configured to press the first cover part (104) in conjunction with attachment of the second cover part (102) and the double-sided adhesive sheet (110); and
the fourth aperture (112) of the second cover part (102), the fifth aperture (1070) of the first cover part (104), the first front recess (1040) of the first cover part (104) and the second front recess (1020) of the second cover part (102) are configured to form a pocket (102A) for the bio-signal processing device (300) to be inserted into and/or removed from.

3. The holder apparatus of claim 2, **characterized in that** the continuous material strip (1024) of the second cover part (102) is configured to be attached with the double-sided adhesive sheet (110) and the continuous material strip (1024) has holes (1024A, 1024B, 1024C) at positions and of sizes corresponding to those of contact electrodes (2002) of the patch electrode structure (200).

4. The holder apparatus of claim 1, **characterized in that** the double-sided adhesive sheet (110) comprises a second aperture (1102) at an area of the first front recess (1040), and the support sheet (1062) is configured to fit inside the second aperture (1102) of the double-sided adhesive sheet (110).

5. The holder apparatus of claim 1, **characterized in that** the fifth aperture (1070) of the first cover part (104) is configured to open to a direction parallel to that of the first aperture (1050).

6. The holder apparatus of claim 1, **characterized in that** the first front recess (1040) of the first cover part (104) is configured to fit to the outer contour of the bio-signal device (300) and cause a frictional contact with the bio-signal processing device (300).

7. The holder apparatus of claim 1, **characterized in that** the holder apparatus (100) comprises adhesive material that causes attachment of at least two of the first cover part (104), the electric connector part (106), the adapter component (108) and the double-sided adhesive sheet (110) together, the adhesive material being cured with optical radiation, and the first cover part (104) being transparent to the optical radiation for the attachment.

8. The holder apparatus of claim 1, **characterized in that** the first cover part (104) comprises a continuous material bridge (1054) between the first aperture (1050) of the first cover part (104) and the gap (1052), and the flexible cable (1064) is configured to continue from the support sheet (1062) though the gap (1052), over the material bridge (1054) toward the first aperture (1050) for a connection with the connector (1060), the flexible cable (1064) and the connector (1060) being partly inside the adapter component (108).

9. The holder apparatus of claim 1, **characterized in that** the adapter component (108) comprises a hole (1084) configured to extend through the adapter component (108), the connector (1060) is partly inside the hole (1080), and the adapter component (108) is configured to connect and seal the connector (1060) and the first cover part (104) together.

10. The holder apparatus of claim 1, **characterized in that** material at least one of, the first cover part (104), the electric connector part (106), and the adapter component (108) being bio-fiber based polymer.

11. The holder apparatus of claim 1, **characterized in that** the adapter component (108) comprises a mating structure (1080) and a leg structure (1082), the leg structure (1082) being configured to support the mating structure (1080), the leg structure (1082) comprises a fifth hole (1084) that is configured receive and keep the connector (1060) at least partly, and the mating structure (1080) is configured to fill the first aperture (1050) of the first cover part (104).

12. A patch electrode structure (200) connectable with the holder apparatus (100) of claim 1, **characterized in that** the patch electrode structure (200) comprises contact electrodes (2002), skin electrodes (2004) and electric conductors (2006) between them, the contact electrodes (2002) being configured to be in electric contact with the holder electrodes (1066) of the support sheet (1062) in response to attachment between the holder apparatus (100) and the patch electrode structure (200) with the double-sided adhesive sheet (110).

13. A method of fabricating a bio-signal arrangement, **characterized by**
forming (500) a first cover part (104) by curving a polymer sheet in a form of a first front recess (1040) and a first rear recess (1042), the first front recess (1040) fitting an outer contour of the bio-signal device (300);
forming (502), to the first cover part (104), a first aperture (1050) at a rear part of the front recess (1040) and a gap (1052) at a rear part of the rear recess (1042);
attaching (504) the first cover part (104) and a double-sided adhesive sheet (110) together such that the concave side is facing the double-sided adhesive sheet (110);
making (506) a front side of the first front recess (1040) of the first cover part (104) open by enclosing a fifth aperture (1070) by the first cover part (104) and the double-sided adhesive sheet (110) in response to attachment of the first cover part (104) and the patch electrode structure (200) together;
inserting (508) a connector (1060) that has flexible conductor cable (1064) through the first aperture (1050) of the first cover part (104);
setting (510) the flexible cable (1064) to go through the gap (1052);
locating a support sheet (1062) that has holder electrodes (1066) connected with the flexible cable (1064) under the concave first cover part (104);
making (512) a mechanical contact between the support sheet (1062) and the connector (1060) by an adapter component (108);
making (514) a mechanical contact between the first cover part (104) and the connector (1060) by the adapter component (108) for support of the connector (1060);
filling (516) the first aperture (1050) with the adapter component (108), the flexible cable (1064) and/or the connector (1060); and
forming (518), by the fifth aperture (1070) of the first cover part (104), and the first front recess (1040) of the first cover part (104), a pocket (102A) for the bio-signal processing device (300) to be inserted into and/or removed from.

14. The fabrication method of claim 13, **characterized by** forming (600) a second cover part (102) by curving a polymer sheet in a form of a second front recess (1020) and a second rear recess (1022);
making (602) a concave side of the second front recess (1020) of the second cover part (102) fit to a convex side of the first front recess (1040) of the first cover part (104);
forming (604) a third aperture (114) at a bottom side of the second front and rear recesses (1020, 1022) of the second cover part (102);
forming (606) a fourth aperture (112) at front side of the second front recess (1020) of the second cover part (102), directing the third and fourth apertures (112, 114) in orthogonal directions, and separating the third and fourth apertures (112, 114) by a continuous material strip (1024) of the second cover part (102);
making (608) a mechanical contact between the adapter component (108) and the second cover part (102) through the first aperture (1050) of the first cover part (104);
performing (610) attachment of the second cover part (102) and the double-sided adhesive sheet (110) together,
making (612) the first cover part (104) to go through the third aperture (114) of the second cover part (102) in conjunction with the attachment,
pressing (614), by the second cover part (102), the first cover part (102) in conjunction with the attachment;
forming (616), by the fourth aperture (112) of the second cover part (102), the fifth aperture (1070) of the first cover part (104), the first front recess (1040) of the first cover part (104) and the second front recess (1020) of the second cover part (102), a pocket (102A) for the bio-signal processing device (300) to be inserted into and/or removed from.

15. The fabrication method of claim 13, **characterized by** forming the second cover part (102) and the first cover part (104) by vacuum forming.
